# EUROPEAN PATENT APPLICATION

(11) **EP 2 255 766 A1**
(43) Date of publication of application: **01.12.2010**
(21) Application number: 09723745.7
(22) Date of filing: 19.01.2009
(51) Int. Cl.: A61F 13/49, A61F 5/44, A61F 13/15, A61F 13/496, A61F 13/511

(54) **DISPOSABLE PANTS-TYPE DIAPER**

(30) Priority: 26.03.2008 JP 2008081757
(71) Applicant: Unicharm Corporation, Ehime 799-0111 (JP)
(72) Inventor: OTSUBO, Toshifumi, Kanonji-shi Kagawa 769-1602 (JP)
(74) Representative: Knights, Rupert
(86) International application number: PCT/JP2009/050615
(87) International publication number: WO 2009/119136

(57) **Abstract**

The present invention aims to provide a disposable pants-type diaper preventing the diaper's skin in the vicinity of external genital and/or in the vicinity of anus from being soiled with body waste.

A crotch region (6) of a disposable pants-type diaper (1) includes a bodily fluid absorbent structure (45) comprising a liquid-pervious inner sheet, a liquid-impervious outer sheet and a bodily fluid absorbent core and, in addition, a body waste retaining space (31) formed by the inner sheet and the innermost sheet member (120) interposed between the inner sheet and the diaper wearer's skin. Segments (33c, 34c) of the innermost sheet member (120) defining peripheral edges (33b, 34b) of a front opening (33) and a rear opening (34) face to each other in a front-back direction (A) and are bonded to each other via two spacers (71, 71) spaced from each other in a transverse direction (B) to form a space (50). A portion of the innermost sheet member (120) extending between the segments (33c, 34c) sags toward the lowermost portion (6a) of the crotch region (6) to form a separator (20a).

## Description

### TECHNICAL FIELD

The present invention relates to disposable pants-type diapers preventing the wearer's skin from being soiled with urine and/or feces.

### RELATED ART

It is well known to provide diapers with a structure serving to prevent urine and feces from being mixed with each other inside the diaper put on the wearer's body.

For example, the diaper described in JP 2002-11044 A (PATENT DOCUMENT 1) has a skin-contact sheet above a liquid-pervious inner sheet covering an absorbent structure wherein, in a crotch region, the skin-contact sheet is formed with an opening and provided with an elastic member attached under tension thereto to surround this opening. In the case of this diaper of prior art, the absorbent structure in the crotch region is formed with a dam-like ridge to block flow of urine and/or feces.

In the case of the disposable pants-type diaper described in JP 2007-296314 A (PATENT DOCUMENT 2), a sheet piece defining a separator serving to prevent urine and feces from being mixed with each other is provided in the crotch region and this sheet piece is fixed along the opposite side edges thereof to the inner surface of the diaper. The sheet piece has a front end and a rear end both extending in the transverse direction of the diaper and these two ends are integrated together on a center line bisecting a width dimension of the diaper.
PATENT DOCUMENT 1: JP 2002-11044 A
PATENT DOCUMENT 2: JP 2007-296314 A

### DISCLOSURE OF THE INVENTION

### PROBLEM TO BE SOLVED BY THE INVENTION

In the case of the diaper described in PATENT DOCUMENT 1, feces move to the inner sheet through the opening of the skin-contact sheet. The skin-contact sheet extends across the crotch region of the diaper in the front-back direction and therefore feces did not properly pass through the opening will flow forward on the skin-contact sheet until the diaper wearer's external genital may be soiled with feces. Furthermore, urine may flow rearward on the skin-contact sheet until the wearer's skin may be soiled with urine in the vicinity of the wearer's anus.

In the case of the diaper described in PATENT DOCUMENT 2, the front end and the rear end of the sheet piece defining the separator are bonded to each other along the center line of the diaper, allowing urine and feces to be separated from each other. With this diaper of prior art, feces would not move forward along the separator. However, considering that the separator for anus and external genital is the integrated front and rear ends, it has sometimes been difficult for this separator to block a flow of loose passage.

In view of the problem as has been described above, it is a principal object of the present invention to improve the disposable pants-type diaper so that the diaper wearer's skin in the vicinity of external genital and/or anus would not be soiled by body waste flowing in the direction from anus to external genital or the inverse direction.

### MEASURE TO SOLVE THE PROBLEM

The object set forth above is achieved, according to the present invention, by an improvement in the disposable pants-type diaper having a front-back direction, a transverse direction and a vertical direction being orthogonal one to another and comprising a crotch region, a front waist region extending forward from the crotch region and a rear waist region extending rearward from the crotch region wherein the crotch region includes a bodily fluid absorbent structure comprising a liquid-pervious inner sheet, a liquid-impervious outer sheet and a bodily fluid absorbent core sandwiched between the inner and outer sheets and curved with the inner sheet inside and the crotch region is additionally formed with a separator adapted to separate urine and feces from each other.

The improvement according to the present invention is characterized in aspects as follow: An innermost sheet member bonded to the inner sheet to be interposed between the pants-type diaper wearer's skin and the inner sheet is adapted to be partially spaced upward from the inner sheet in the vertical direction and thereby to form a body waste retaining space having a front opening allowing urine to flow into the body waste retaining space and a rear opening allowing feces to move into the body waste retaining space. The innermost sheet member is bonded to itself in regions thereof defining at least respective segments of peripheral edges of the front opening and the rear opening and opposed to each other in the front-back direction via a pair of spacers spaced from each other in the transverse direction to define a space having a required dimension. A portion of the innermost sheet member extending between those segments opposed to each other in the front-back direction sags toward the lowermost portion of the crotch region to form the separator.

According to one preferred embodiment of the present invention, each of the two spacers is formed of a flexible and elastic material.

According to another preferred embodiment of the present invention, each of the two spacers has a dimension in a range of 1 to 30mm in the front-back direction and is spaced from each other by a distance in a range of 5 to 70mm in said transverse direction.

### EFFECT OF THE INVENTION

In the disposable pants-type diaper according to the present invention, the innermost sheet member forming the separator adapted to separate urine and feces from each other and thereby to prevent urine and feces from being mixed with each other is bonded to itself via the spacers along a segment of the peripheral edge of the front opening and a segment of the peripheral edge of the rear opening both for the body waste retaining space to form the space of a required dimension. The portion of the innermost sheet member extending in the front-back direction between these segments of the respective openings' peripheral edges sags toward the lowermost portion of the crotch region. The portion of the innermost sheet member sagging in this manner serves as the barrier functioning to prevent urine excreted on the front half of the crotch region and feces excreted on the rear half of the crotch region from being mixed with each other. With the pants-type diaper put on the wearer's body, the segments of the respective openings' peripheral edges defining the space therebetween are put in close contact with the wearer's crotch skin and thereby serve as the barriers against urine flowing rearward and loose passage flowing forward along the wearer's skin. In consequence, the wearer's skin in the vicinity of anus would not be soiled with urine and the wearer's skin in the vicinity of external genital would not be soiled with feces.

### BRIEF DESCRIPTION OF THE DRAWINGS

[FIG. 1] Fig. 1 is a partially cutaway perspective view of a pants-type diaper according to the present invention.
[FIG. 2] Fig. 2 is a plan view showing the pants-type diaper having front and rear waist regions thereof unsealed from each other and flatly developed.
[FIG. 3] Fig. 3 is a sectional view taken along the line III-III in Fig. 1.
[FIG. 4] Fig. 4 is an overhead view of the pants-type diaper as viewed from above in a direction indicated by the arrows IV-IV in Fig. 3.
[FIG. 5] Fig. 5 is a partially cutout perspective view of one preferred embodiment of the pants-type diaper according to the present invention.
[FIG. 6] Fig. 6 is a sectional view taken along the line VI-VI in Fig. 5.

### IDENTIFICATION OF REFERENCE NUMERALS USED IN THE DRAWINGS

- 1: pants-type diaper
- 2: inner sheet
- 3: outer sheet
- 4: absorbent core
- 6: crotch region
- 6a: lowermost portion of crotch region
- 7: front waist region
- 8: rear waist region
- 10: chassis
- 12: leg-openings
- 13: peripheries of leg-openings
- 20: innermost sheet member
- 20a: separator
- 21: portion of innermost sheet member (front end)
- 22: portion of innermost sheet member (rear end)
- 31: body waste retaining space
- 33: front opening
- 33b: peripheral edge
- 33c: segment (lower segment)
- 34: rear opening
- 34b: peripheral edge
- 34c: segment (lower segment)
- 36: portion extending in front-back direction
- 45: liquid-absorbent structure
- 50: void space
- 71: spacers
- 120: sheet member (innermost sheet member)
- A: front-back direction
- B: transverse direction
- C: vertical direction

### DETAILED DESCRIPTION OF THE PREFERRED EMBODIMENTS

Details of the disposable pants-type diaper according to the present invention will be more fully understood from the description given hereunder with reference to the accompanying drawings.

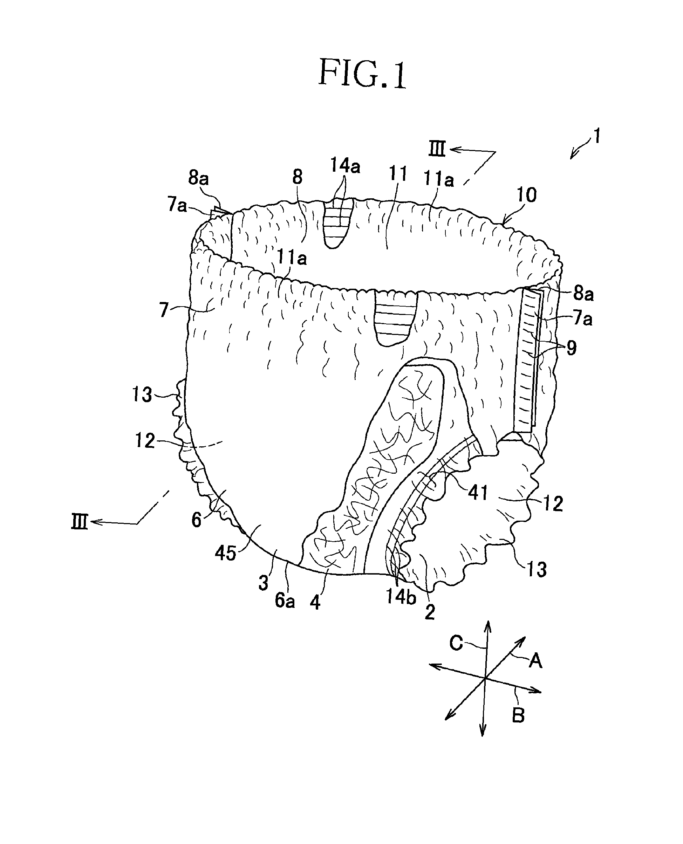

Fig. 1 is a partially cutaway perspective view showing the disposable pants-type diaper 1 as put on the wearer's body wherein a front-back direction, a transverse direction and a vertical direction of the diaper 1 are respectively indicated by double-headed arrows A, B and C which are orthogonal one to another. The pants-type diaper 1 includes a pant-shaped chassis 10 comprising a liquid-pervious inner sheet 2, a liquid-impervious outer sheet 3 and a bodily fluid-absorbent core 4. The chassis 10 has a crotch region 6, a front waist region 7 extending forward from the crotch region 6 and a rear waist region 8 extending rearward from the crotch region 6. The front and rear waist regions 7, 8 are put flat and sealed together along respective pairs of side edges 7a, 7a and 8a, 8a of these waist regions 7, 8, more specifically, at joints 9 arranged intermittently in the vertical direction as viewed in Fig. 1 along these side edges 7a, 7a and 8a, 8a and thereupon a waist-opening 11 and a pair of leg-openings 12 are formed. Along a periphery 11a of the waist-opening 11, a plurality of waist elastic members 14a extending in a circumferential direction is sandwiched between the inner and outer sheets 2, 3 and bonded under tension to at least one of the inner and outer sheets 2, 3. Along a periphery of each of the leg-openings 12, a plurality of leg elastic members 14b extending in the circumferential direction is sandwiched between the inner and outer sheets 2, 3 and bonded under tension to at least one of these inner and outer sheets 2, 3. Three components, i.e., the leg elastic members 14b and the inner and outer sheets 2, 3 cooperate together to define respective annular elastic regions 41 surrounding the wearer's legs. Of the pants-type diaper 1, at least the crotch region 6 includes a bodily fluid absorbent structure 45 comprising the inner and outer sheets 2, 3 and the absorbent core 4 sandwiched therebetween. The bodily fluid absorbent structure 45 extends in the front-back direction A and curves convexly downward to define a bottom portion 6a of the crotch region 6.

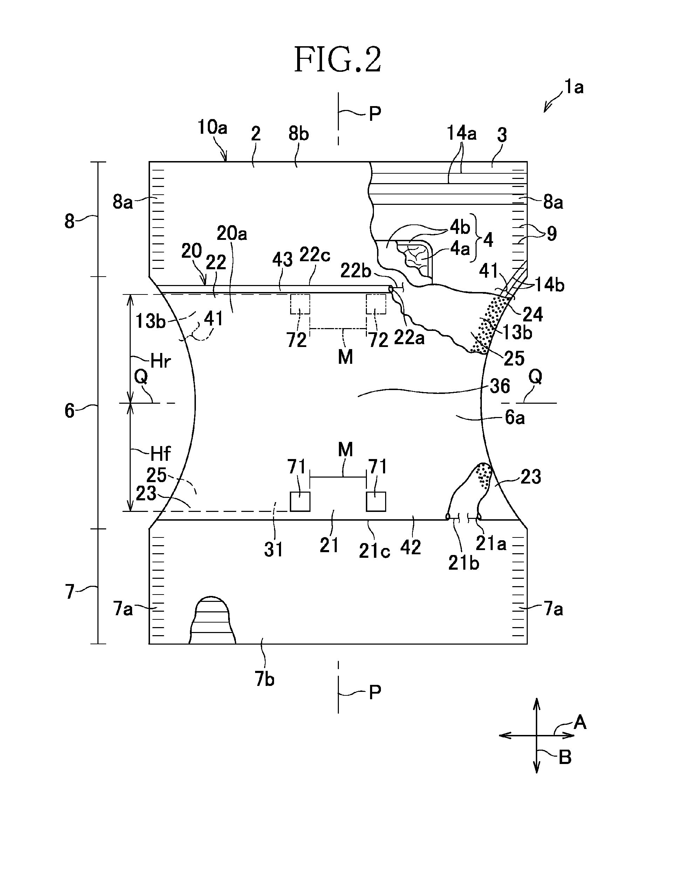

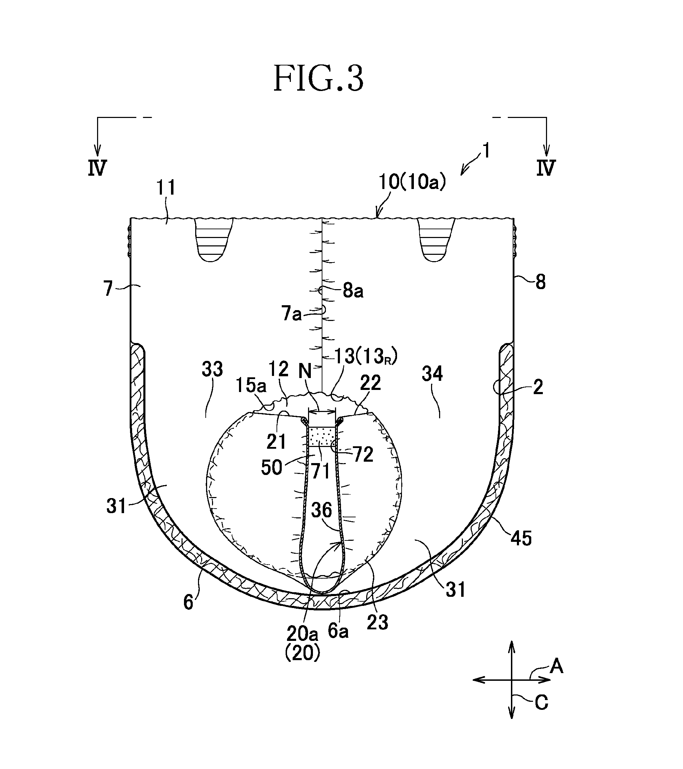

Fig. 2 is a partially cutaway perspective view showing the pants-type diaper 1a as after the front and rear waist regions 7, 8 of the diaper 1 have been unsealed at the joints 9 and flatly developed as a whole in the front-back direction A as well as in the transverse direction B wherein such developed diaper 1a is shown as viewed from its inner side. In the state of such developed diaper 1a, the chassis 10 in Fig. 1 appears as a concave shaped flattened chassis 10a. The periphery 11a of the waist-opening 11 in Fig. 1 appears herein as a front end 7b and a rear end 8b of the flattened chassis 10a and the peripheries 13 of the respective leg-openings 12 appear herein as the crotch region's side edges 13b of the flattened chassis 10a. While the crotch region's side edges 13b convexly curve toward a center line P-P extending in the front-back direction A to bisect a dimension of the flattened chassis 10a in the transverse direction X, the respective pairs of side edges 7a, 7a and 8a, 8a of the front and rear waist regions 7, 8 extend generally in parallel to this center line P-P. The absorbent core 4 also is concave shaped and is formed of a mixture 4a of fluff pulp fibers and super-absorbent polymer particles wrapped with a bodily fluid-absorbent and highly dispersant wrapping sheet 4b such as a tissue paper. In the flattened chassis 10a, the innermost sheet member 20 made of hydrophobic sheet material, more preferably, hydrophobic and liquid-impervious sheet material is attached to inner sheet 2 defining the inner surface of the flattened chassis 10a in the crotch region 6 so that the innermost sheet member 20 may define a separator 20a (See Fig. 3). Such flatly developed diaper 1a is formed symmetrically about the center line P-P extending in the front-back direction A. The lowermost portion 6a of the crotch region 6 lies on a transverse center line Q-Q bisecting a dimension of the flatly developed diaper 1a in the front-back direction A.

The innermost sheet member 20 is attached to the flattened chassis 10a so that the innermost sheet member 20 may be interposed between the inner sheet 2 and the diaper wearer's skin (not shown) when the pants-type diaper 1 is put on the wearer's body. The innermost sheet member 20 has side edges 23 respectively fixed to the crotch region's side edges 13b by hot melt adhesive 24, a front end 21 lying before the lowermost portion 6a of the crotch region 6 and extending in the transverse direction B between the side edges 23, 23 and a rear end 22 lying behind the lowermost portion 6a of the crotch region 6 and extending in the transverse direction B between the side edges 23, 23. The innermost sheet member 20 in the illustrated embodiment is not bonded to the inner sheet 2 and adapted to be spaced from the inner sheet 2 except the side edges 23 so that a tunnel- or pocket-like body waste retaining space 31 extending from the front end 21 to the rear end 22 may be defined between the innermost sheet member 20 and the inner sheet 2. Along the front end 21 and the rear end 22, the innermost sheet member 20 is folded back to form a sleeve 21a and a sleeve 22a, respectively. These sleeves 21a, 22a contain therein a front elastic member 21b and a rear elastic member 22a attached under extension thereto, respectively, to define front elastic region 42 and a rear elastic region 43 both extending between the crotch region's side edges 13b. These elastic regions 42, 43 intersect with the elastic regions 41 of the flattened chassis 10a. A distance Hf from the transverse center line Q-Q to the front end 21 is generally equal to a distance Hr from the transverse center line to the rear end 22.

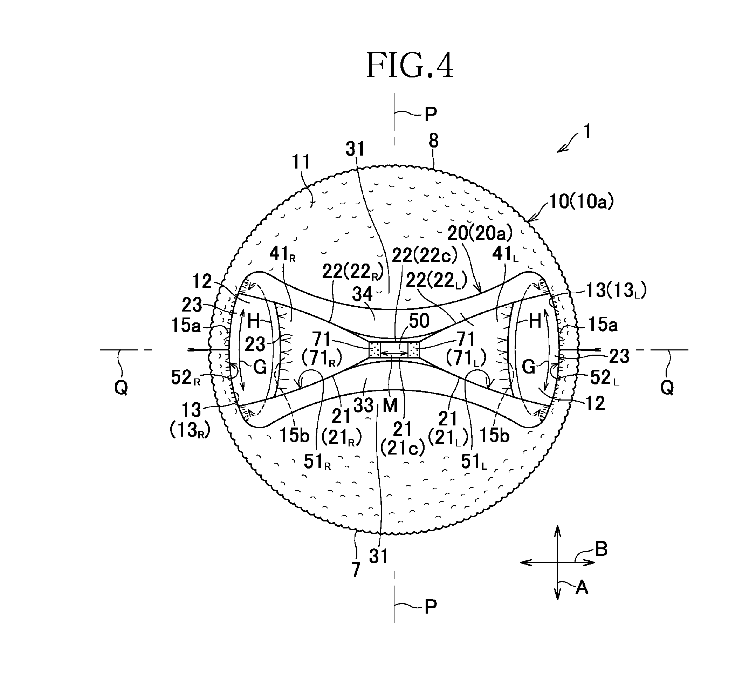

The innermost sheet member 20 is provided on the front end 21 with a pair of spacers 71 located symmetrically about the center line P-P extending in the front-back direction A and spaced from each other by a dimension M. Each of the spacers 71 may be provided in the form of a block made of a sponge, a foamed polyurethane, a foamed polystyrene, a foamed polyethylene, a felt or a nonwoven fabric each having a flexibility sufficient to be easily deformed under a body weight of the wearer of the pants-type diaper 1 and, more preferably, having an elastic recovery properties also sufficient for a rapid recovery after deformation. The innermost sheet member 20 is provided on the rear end 22 with a pair of regions 72 indicated by imaginary lines which are also located symmetrically about the center line P-P extending in the front-back direction A and spaced from each other by a dimension M in the transverse direction B. The pair of spacers 71 are bonded to the innermost sheet member 20 at the pair of regions 72 indicated by the imaginary lines as the side edges 7a, 7a and 8a, 8a of the flattened chassis 1a are joined together to obtain the pants-type diaper 1 (See Figs. 3 and 4).

Fig. 3 is a sectional view taken along the line III-III in Fig. 1 and the line III-III corresponds to the center line P-P extending in the front-back direction A in Fig. 2. In the pant-shaped flattened chassis 10, the front and rear waist regions 7, 8 are joined together along the respective side edges 7a, 7a and 8a, 8a thereof. The crotch region 6 curves in U-shape as viewed in the front-back direction A and the lowermost portion 6a of the crotch region 6 defines a bottom of this U-shaped curve. The crotch region's side edges 13b appear in Fig. 3 as the peripheral edges 13 defining the leg-openings 12. The innermost sheet member 20 has the front end 21 and the rear end 22 thereof bonded to each other via the spacers 71 and the portion 36 shown in Fig. 2 to extend in the front-back direction A between the front end 21 and the rear end 22 sags toward the lowermost portion 6a to form the separator 20a partitioning the crotch region 6 into a front half and a rear half. Each of the spacers 71 has a dimension N in the front-back direction A and is attached to the innermost sheet member 20 by adhesion or sealing. The presence of the spacers 71 allows the front end 21 and the rear end 22 to leave between them a space 50 (See Fig. 4) having a dimension N along the center line P-P extending in the front-back direction A. The regions in which the spacers 71 are joined to the rear end 22 are designated by a reference numeral 72. The separator 20a may be formed so that the portion 36 of the innermost sheet member 20 is put close to, or in contact with or joined to the lowermost portion 6a or the inner sheet 2 in the vicinity of the portion 6a. Fig. 3 exemplarily shows one of these three manners of implementation in which the portion 36 of the innermost sheet member 20 is joined to the lowermost portion 6a or the inner sheet 2 in the vicinity of the portion 6a. In the body waste retaining space 31 formed by cooperation of the innermost sheet member 20 with the inner sheet 2, the front end 21 is spaced from the inner sheet 2 to define a front opening 33 and the rear end 22 is spaced from the inner sheet 2 to define a rear opening 34.

Fig. 4 is an overhead view of the pants-type diaper 1 of Fig. 1 as viewed from above the waist-opening 11 in a direction indicated by the arrows IV-IV in Fig. 3. As viewed in the transverse direction B of the pants-type diaper 1, the front end 21 and the rear end 22 of the innermost sheet member 20 respectively have central segments 21c, 22c defined between a pair of the spacers 71, right-hand segments 21_{R}, 22_{R} adapted to be put in contact with a right leg of the diaper wearer (not shown) and left-hand segments 21_{L}, 22_{L} adapted to be put in contact with a left leg of the diaper wearer. The central segments 21c, 22c extend in parallel to each other in the transverse direction B to form a space having a dimension N in the front-back direction A and a dimension M in the transverse direction B. These central segments 21c, 22c are adapted to come in contact with the diaper wearer's skin between his or her external genital and anus as the pants-type diaper 1 is put on the wearer's body. The right-hand segments 21_{R}, 22_{R} cooperate with each other to define a laterally facing V-shaped space so that the wearer's right leg may be guided through this laterally facing V-shaped space. In a similar manner, the left-hand segments 21_{L}, 22_{L} cooperate with each other to define a laterally facing V-shaped space so that the wearer's left leg may be guided through this laterally facing V-shaped space. As illustrated, the respective leg-openings 12 of the chassis 10 have a right leg peripheral edge 13_{R} and a left leg peripheral edge 13_{L}. The respective peripheral edges 13_{R}, 13_{L} comprise upper segments 15a not overlapping the side edges 23 of the innermost sheet member 20 and lower segments 15b overlapping the side edges 23 and bonded thereto (See Fig. 3 also). In Fig. 4, respective ranges of these upper segments 15a and lower segments 15b are indicated by double-headed arrows G and H.

Sequence in which the pants-type diaper 1 may be put on the wearer's body will be described below together with behavior of the pants-type diaper. First, the front and rear waist regions 7, 8 of the chassis 10 may be spaced from each other in the front-back direction A and then the waist-opening 11 may be sufficiently broadened as seen in Figs. 1 and 4. Thereby the front end 21 of the separator 20a formed of the innermost sheet member 20 is deformed so that the right-hand segment 21_{R} and the left-hand segment 21_{L} are curved in V-shape facing forward in the front-back direction A around a pair of the spacers. In a similar manner, the rear end 22 is deformed so that the right-hand segment 22_{R} and the left-hand segment 22_{L} are curved in V-shape facing rearward in the front-back direction A (See Fig. 4). Consequently, the front opening 33 and the rear opening 34 for the body waste retaining space 31 are automatically broadened (See Fig. 3). The left-hand segments 21_{L}, 22_{L} are spaced from each other in the front-back direction A at the same moment that the right-hand segments 21_{R}, 22_{R} are spaced from each other in the front-back direction A. The respective central segments 21c, 22c of the front end 21 and the rear end 22 cooperate together to define the space 50. Now the diaper wearer (not shown) may guide his or her right leg into an opening 41_{R} for the right leg defined by the upper segment 15a of the right leg peripheral edge 13_{R}, the right-hand segment 21_{R} of the front end 21 and the right-hand segment 22_{R} of the rear end 22. Then the wearer may guide his or her right leg into the leg-opening 12 for the right leg defined by the upper segment 15a and the lower segment 15b of the right leg peripheral edge 13_{R}. Without interruption, the wearer may guide his or her left leg into an opening 41_{L} for the left leg defined by the upper segment 15a of the left leg peripheral edge 13_{L}, the left-hand segment 21_{L} of the front end 21 and the left-hand segment 22_{L} of the rear end 22. Then the wearer may guide his or her right leg into the leg-opening 12 for the left leg defined by the upper segment 15a and the lower segment 15b of the left leg peripheral edge 13_{L}.

In the pants-type diaper 1 after it has been put on the wearer's body, the peripheral edges 13 of the respective leg-openings 12, i.e., the right leg peripheral edge 13_{R} and the left leg peripheral edge 13_{L} are elastically contractible in a circumferential direction thereof, on one hand, and the front end 21 and the rear end 22 of the separator 20a are elastically contractible in the transverse direction B, on the other hand. With such arrangement, the upper segment 15a of the peripheral edge 13_{R} for the right leg and the right-hand segments 21_{R}, 22_{R} of the separator 20a are elastically put in close contact around the right leg in the vicinity of the right leg's inguinal region to form a primary seal 51_{R} (See Fig. 4) serving to prevent leak of bodily fluids. Below the primary seal 51_{R}, the lower segment 15b and the upper segment 15a of the peripheral edge 13_{R} for the right leg are integrated to form a secondary seal 52_{R} (See Fig. 4) serving to prevent leakage of bodily fluids. Function of this secondary seal 52_{R} is similar to that of the leg seal in the conventional pants-type diaper. The arrangement and function as have been described just above are also true with respect to the left leg. Specifically, the upper segment 15a of the peripheral edge 13_{L} for the left leg and the left-hand segments 21_{L}, 22_{L} of the separator 20a are elastically put in close contact around the left leg in the vicinity of the left leg's inguinal region to form a primary seal 51_{L} serving to prevent leakage of bodily fluids. Below the primary seal 51_{L}, the lower segment 15b and the upper segment 15a of the peripheral edge 13_{L} for the left leg are integrated to form a secondary seal 52_{L} serving to prevent leak of bodily fluids. The pants-type diaper 1 having been put on the wearer's body may be sufficiently pulled up along the body to assure that the central segments 21c, 22c in the separator 20a come in contact with the wearer's skin between the wearer's external genital and anus.

With the pants-type diaper 1 put on the wearer's body in the state as has been described above, the portion 36 of the separator 20a is positioned to divide the wearer's crotch region into a front half and a rear half. In the front half of the wearer's crotch region, the wearer's external genital lying before the front end 21 is exposed in the front opening 33 and, in the rear half of the wearer's crotch region, the wearer's anus lying behind the rear end 22 is exposed in the rear opening 34. Consequentially, it is assure that urine discharged by the wearer flows through the front opening 33 into the pocket-like body waste retaining space 31 and feces excreted by the wearer moves through the rear opening 34 into the body waste retaining space 31. In this way, it is possible for the pants-type diaper 1 to prevent urine and/or feces from coming in contact with the wearer's skin by the presence of the separator 20a. In addition, the central segments 21c, 22c of the separator 20a is kept spaced from each other in the font-back direction A to define dual barriers adapted to prevent urine from flowing along the wearer's skin toward the anus and simultaneously to define dual barriers adapted to prevent loose passage from flowing along the wearer's skin toward the wearer's external genital as long as these central segments 21c, 22c are kept in contact with the wearer's skin. Furthermore, even if these central segments 21c, 22c are not kept in contact with the wearer's skin, these central segments 21c, 22c are adapted to prevent loose passage flowing back from the body waste retaining space through the rear opening 34 to the wearer's external genital, for example, when the wearer sits flat on the floor unless the loose passage flows beyond the space 50. In other words, with the pants-type diaper 1 the wearer's skin in the vicinity of the anus would not be stained with urine and wearer's skin in the vicinity of the external genital would not be soiled with loose passage. Urine and feces once retained in the body waste retaining space 31 can no more flow on the inner sheet 2 in the front-back direction A and be mixed with each other since the separator 20a is kept close to or in contact with or bonded to the inner sheet 2 at the lowermost portion 6a of the crotch region 6. Therefore fluidity of feces would not increase due to mixture with urine and the wearer's skin would not be readily soiled with such loose passage. In addition, for the wearer's legs, the primary seals 51_{R}, 51_{L} and the secondary seals 52_{R}, 52_{L} are formed to prevent urine and/or feces from readily leaking out of the pants-type diaper 1 even if urine and/or feces do not flow into the body waste retaining space 31 but flowing along the wearer's legs.

In the pants-type diaper 1, the front end 21 of the innermost sheet member 20 defining the separator 20a partially defines the peripheral edge of the front opening 33. As viewed in the front-back direction A, the front end 21 lies behind the wearer's external genital and positioned so that the external genital can be reliably exposed in the front opening 33. While the front end 21 is exemplarily illustrated to extend linearly in the transverse direction B, it is also possible to form the front end 21 to curve convexly toward the transverse center line Q-Q. The rear end 22 of the innermost sheet member 20 partially defines the peripheral edge of the rear opening 34. As viewed in the front-back direction A, the rear end 22 lies before the wearer's anus and positioned so that the anus can reliably face the rear opening 34. While such rear end 22 is exemplarily illustrated to extend linearly in the transverse direction B, it is possible to form the rear end 22 to curve convexly toward the transverse center line Q-Q.

For the present invention, the shape of the spacers 71 defining the space 50 is not specified in particular. While the dimension N of the spacer 71 is not specified but depends on whether the pants-type diaper 1 is for baby or for adult, for comprehensive application, the dimension N is preferably in a range of 1 to 30mm and more preferably in a range of 1 to 5mm. The dimension N exceeding 30mm will progressively make it difficult to expose the wearer's external genital in the front opening 33 and to expose the wearer's anus in the rear opening 34. A distance dimension M between a pair of the spacers 71 is preferably in a range of 5 to 70mm and more preferably in a range of 25 to 30mm. The distance dimension M smaller than 5mm may sometimes make it difficult for body waste to pass through the space 50 and the distance dimension M exceeding 70mm may sometimes unacceptably broaden the width of the crotch region 6.

In the crotch region 6 of the flattened chassis 10a shown by Fig. 2, the inner and outer sheets 2, 3 put flat together define side flaps 25 outboard of the liquid-absorbent core 4. When the crotch region 6 is curved in U-shape under contraction of the elastic regions 41 and thereupon the pants-type diaper 1 is put into the state as illustrated by Fig. 1, the side flaps 25 behave to raise themselves along side edges 4c of the core 4 in the vicinity of the transverse center line Q-Q, i.e., in the vicinity of the lowermost portion 6a of the crotch region 6. The innermost sheet member 20 having the side edges 23 bonded to the inner surface of the elastic regions 41 pulls the side flaps 25 inward in the transverse direction of the pants-type diaper 1, i.e., toward the center line P-P extending in the front-back direction as the waist-opening 11 is broadened. In consequence, the behavior of the side flaps 25 to raise themselves is further enhanced. Such behavior of the side flaps 25 becomes further significant as the front end 21 and the rear end 22 elastically contract. The side flaps 25 having such behavior come in close contact with the wearer's thighs from below with respect to the pants-type diaper 1 and thereby effectively prevent bodily fluids from leaking out along the wearer's thighs.

To implement the present invention, liquid-pervious sheet material such as nonwoven fabric or perforated plastic film may be used as the inner sheet 2. Liquid-impervious sheet material such as plastic film or composite sheet consisting of plastic film and nonwoven fabric may be used as the outer sheet 3. Fluff pulp fibers or a mixture of fluff pulp fibers and super-absorbent polymer particles wrapped with an appropriate sheet material such as a tissue paper may be used as the liquid-absorbent core 4. A hydrophobic sheet material such as a nonwoven fabric or plastic film, more preferably, a hydrophobic and liquid-impervious sheet material may be used as the innermost sheet member 20. While the innermost sheet member 20 are preferably provided along the front end 21 and the rear end 22 with the elastic members 21b, 22b attached under tension thereto so that the innermost sheet member 20 may be elastically contracted, it is possible to eliminate use of the elastic members 21b, 22b by using an elastic sheet material adapted to be elastically contracted in the transverse direction B as the innermost sheet member 20. It should be appreciated that the present invention includes an embodiment according to which both the front end 21 and the rear end 22 are not elastically contractible in the transverse direction B.

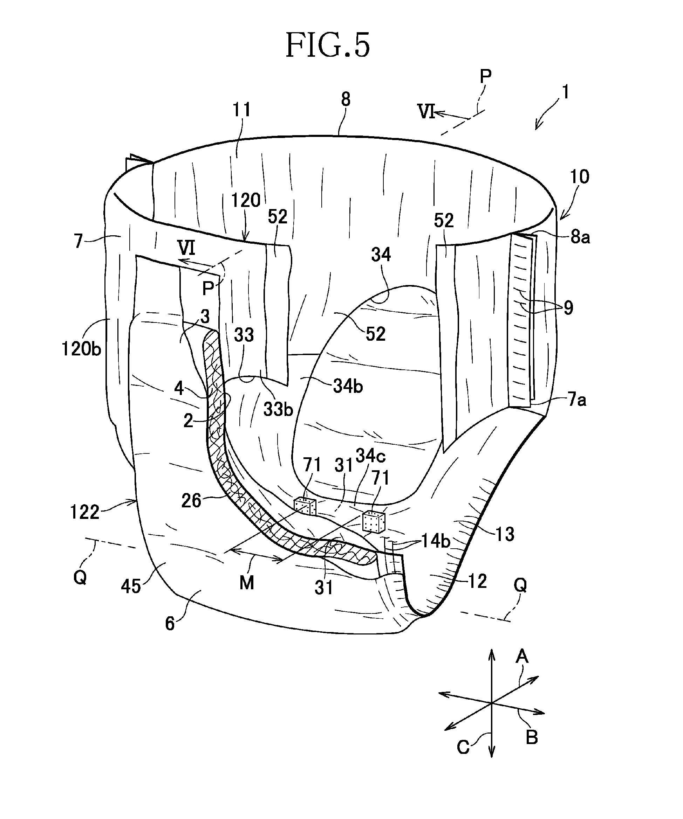

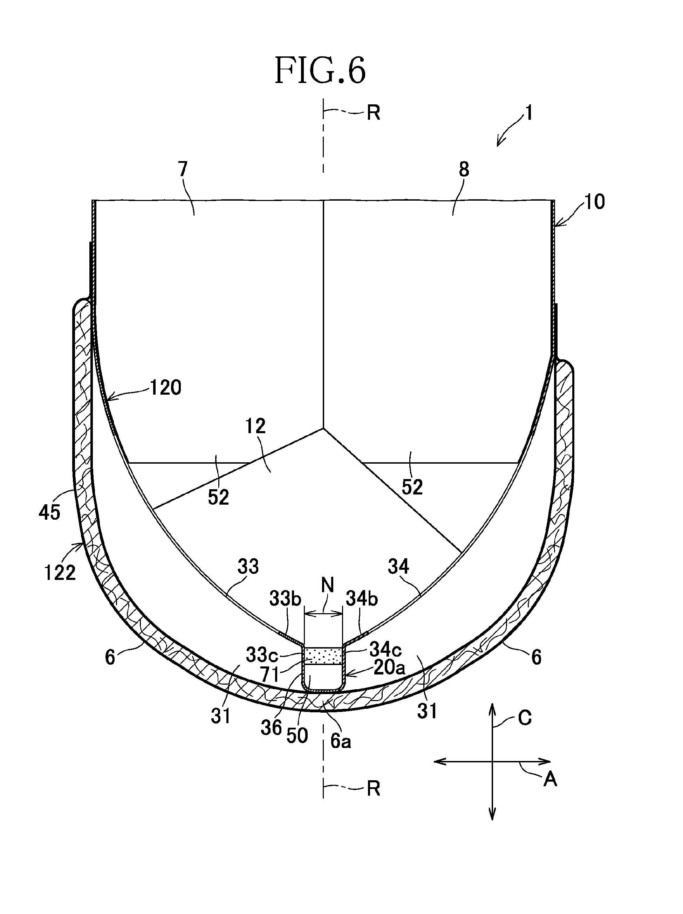

Fig. 5 is a partially cutaway perspective view of one preferred embodiment of the pants-type diaper 1 according to the present invention. Regions and members of this embodiment similar to those in the pants-type diaper 1 of Fig. 1 are designated with similar reference numerals. Specifically, the pants-type diaper 1 of Fig. 5 also has the crotch region 6, the front waist region 7 and the rear waist region 8 wherein the side edges 7a, 7a and 8a, 8a of the front and rear waist regions 7, 8 are put flat and sealed together at the joints 9 arranged intermittently in the vertical direction to form the waist-opening 11 and a pair of the leg-openings 12. The pants-type diaper 1 according to this embodiment also includes the innermost sheet member 120 facing the wearer' skin (not shown) and a bodily fluid absorbent member 122 attached to the outer surface 120b of the innermost sheet member 120 wherein these innermost sheet member 120 and the bodily fluid absorbent member 122 are integrated with each other to form the pant-shaped chassis 10. The innermost sheet member 120 is preferably hydrophobic, more preferably hydrophobic and liquid-impervious. Elasticized sheet pieces 52 are bonded under tension to the inner surface of the innermost sheet member 120 in the front waist region 7 and the rear waist region 8, respectively, so that the innermost sheet member 120 may be elastically contracted in the circumferential direction. The innermost sheet member 120 is formed in the crotch region 6 with the front opening 33 and the rear opening 34. The front opening 33 has the peripheral edge 33b and is positioned in front of the transverse center line Q-Q so that the wearer's external genital can be exposed in this front opening 33. The rear opening 34 has the peripheral edge 34b and is positioned behind the transverse center line Q-Q so that the wearer's anus can be exposed in this rear opening 34. Referring to Fig. 5, a pair of spacers 71 are attached to the peripheral edge 34b by which the innermost sheet member 120 is joined to itself along a lower segment 33c of the peripheral edge 33b and a lower segment 34c of the peripheral edge 34b (See Fig. 6 also).

The bodily fluid absorbent member 122 has the bodily fluid absorbent structure 45 comprising the liquid-pervious inner sheet 2, the liquid-impervious outer sheet 3 and the bodily fluid absorbent core 4 sandwiched between the inner and outer sheets 2, 3. These inner and outer sheets 2, 3 extend outward beyond the periphery of the core 4 and put flat and bonded together outside the periphery of the core 4 wherein at least one of these inner and outer sheets 2, 3 is bonded to the innermost sheet member 120 so that the front opening 33 and the rear opening 34 may be covered with said one of the inner and outer sheets 2, 3 from outside of the innermost sheet member 120. Along the side edges 13 of the crotch region 6, the inner sheet 2, the outer sheet 3 and the innermost sheet member 120 are placed one on another and bonded together. Also along the side edges 13, the leg elastic members 14b sandwiched between the inner sheet 2 and the outer sheet 3 is bonded under tension to at least one of these two sheets 2, 3. Between the side edges 13, the inner sheet 2 and the innermost sheet member 120 are not bonded to each other so that the innermost sheet member 120 may be spaced from the inner sheet 2 in the thickness direction of the core 4 to form the body waste retaining space 31 into which urine flows through the front opening 3 and feces moves through the rear opening 34.

Fig. 6 is a sectional view taken along the line VI-VI in Fig. 1 wherein the line VI-VI corresponds to the center line P-P extending in the front-back direction. The innermost sheet member 120 is bonded to itself along the lower segment 33c of the peripheral edge 33b defining the front opening 33 and the lower segment 34c of the peripheral edge 34b defining the rear opening 34 to form the space 50 having the dimension N in the front-back direction A between these two segments 33c, 34c. The space 50 has the dimension M in the transverse direction B (See Fig. 5). The portion 36 of the innermost sheet member 120 defined in the front-back direction A between the lower segments 33c, 34c sags toward the lowermost portion 6a of the crotch region 6. The portion 36 of the innermost sheet member 120 may be put close to, or in contact with or joined to the lowermost portion 6a or the inner sheet 2 in the vicinity of the portion 6a. Fig. 6 exemplarily shows one of these three manners of implementation in which the portion 36 of the innermost sheet member 120 is joined to the lowermost portion 6a or the inner sheet 2 in the vicinity of the portion 6a.

With the pants-type diaper 1 implemented as illustrated by Fig. 5 put on the wearer's body, the lower segments 33c, 34c function as dual barriers one of which prevents urine from flowing rearward along the wearer's skin in crotch region and the other of which prevents loose passage from flowing forward along the wearer's skin in the crotch region so long as the lower segments 33c, 34c are kept in contact with the wearer's skin in the crotch region. In this way, also with the pants-type diaper 1 of Fig. 5, the wearer's skin in the vicinity of the anus would not be soiled with urine and the wearer's skin in the vicinity of the external genital would not be soiled with loose passage. The innermost sheet member 120 sags toward the lowermost portion 6a of the crotch region 6 and thereby divides the body waste retaining space 31 into front and rear halves to define the separator 20a adapted to prevent urine and feces from being mixed together on the inner sheet 2. The lower segments 33c, 34c in the pants-type diaper 1 of Fig. 5 provide the function equivalent to that of the front edge 21 and the rear edge 21 of the innermost sheet member 20 in the pants-type diaper 1 of Fig. 1.

## Claims

1. A disposable pants-type diaper having a front-back direction, a transverse direction and a vertical direction being orthogonal one to another and comprising a crotch region, a front waist region extending forward from said crotch region and a rear waist region extending rearward from said crotch region wherein said crotch region includes a bodily fluid absorbent structure comprising a liquid-pervious inner sheet, a liquid-impervious outer sheet and a bodily fluid absorbent core sandwiched between said inner and outer sheets and curved with inner sheet inside and said crotch region is additionally formed with a separator adapted to separate urine and feces from each other, said disposable pants-type diaper being **characterized in that:**
an innermost sheet member bonded to said inner sheet to be interposed between a pants-type diaper wearer's skin and said inner sheet is adapted to be partially spaced upward from said inner sheet in said vertical direction and thereby to form a body waste retaining space having a front opening allowing urine to flow into said body waste retaining space and a rear opening allowing feces to move into said body waste retaining space;
said innermost sheet member is bonded to itself in segments thereof defining at least respective parts of peripheral edges of said front opening and said rear opening and opposed to each other in said front-back direction via a pair of spacers spaced from each other in said transverse direction to define a space having a required dimension; and
a portion of said innermost sheet member extending between said segments opposed to each other in said front-back direction sags toward a lowermost portion of said crotch region to form said separator.

2. The pants-type diaper defined by Claim 1 wherein each of said two spacers is formed of a flexible and elastic material.

3. The pants-type diaper defined by Claim 1 or 2 wherein each of said two spacers has a dimension in a range of 1 to 30mm in said front-back direction and is spaced from each other by a distance in a range of 5 to 70mm in said transverse direction.
